# EUROPÄISCHE PATENTSCHRIFT

(11) **EP 0 954 440 B1**
(45) Veröffentlichungstag und Bekanntmachung des Hinweises auf die Patenterteilung: **22.10.2003**
(21) Anmeldenummer: 98901336.2
(22) Anmeldetag: 03.01.1998
(51) Int. Cl.: B32B 27/36, B32B 29/00, A61K 9/70, C09J 7/02, G09F 3/10

(54) **ABLÖSBARE SCHUTZFOLIE FÜR WIRKSTOFFHALTIGE, INSBESONDERE SELBSTKLEBENDE PFLASTERSYSTEME**
REMOVABLE PROTECTIVE FILM FOR PLASTER SYSTEMS, IN PARTICULAR SELF-ADHESIVE PLASTER SYSTEMS, CONTAINING ACTIVE SUBSTANCES
FEUILLE PROTECTRICE DETACHABLE POUR SYSTEMES D'EMPLATRE, EN PARTICULIER AUTO-ADHESIFS, RENFERMANT DES MATIERES ACTIVES

(30) Priorität: 23.01.1997 DE 19702314
(43) Veröffentlichungstag der Anmeldung: 10.11.1999
(73) Patentinhaber: LTS LOHMANN Therapie-Systeme GmbH, 56567 Neuwied (DE)
(72) Erfinder: MÜLLER, Walter, D-56564 Neuwied (DE)
(74) Vertreter: Flaccus, Rolf-Dieter, Dr.
(86) Internationale Anmeldenummer: EP9800012
(87) Internationale Veröffentlichungsnummer: WO98032604

(56) Entgegenhaltungen:
- EP-A- 0 451 202
- EP-A- 0 536 766
- EP-A- 0 637 618
- FR-A- 2 498 986
- US-A- 4 904 247
- DATABASE WPI Section Ch, Week 8925 Derwent Publications Ltd., London, GB; Class A28, AN 89-182875 XP002064465 & JP 01 121 388 A (MITSUBISHI PAPER MILLS LTD) , 15.Mai 1989

## Beschreibung

Wirkstoffhaltige Pflastersysteme zur Abgabe von Wirkstoffen, insbesondere zur Abgabe von medizinischen Wirkstoffen an bzw. durch die menschliche Haut gewinnen aufgrund ihrer therapeutischen Vorteile zunehmend an Bedeutung. Man kann sie grob in zwei Klassen unterteilen, nämlich in die transdermalen und die topischen Pflastersysteme. Bei den transdermalen Systemen wird der Wirkstoff durch die menschliche oder tierische Haut geschleust, wobei dieser Wirkstoff dann systemisch zur Wirkung kommt. Bei topischen Pflastersystemen wird angestrebt, den Wirkstoff bevorzugt nur im Gewebe unterhalb der Applikationstelle zur Wirkung kommen zu lassen.

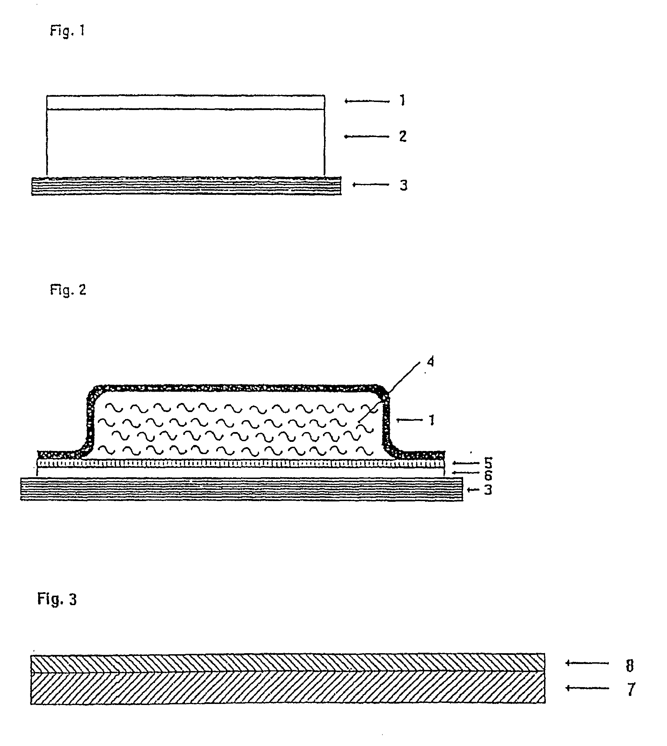

Beide Pflastersysteme sind zur Befestigung auf der Haut zumindest zum Teil selbstklebend ausgerüstet. Im einfachsten Fall bestehen solche Systeme (Fig.1) aus einer Rückschicht, einer selbstklebenden Schicht mit darin eingearbeitetem Wirkstoff und einer ablösbaren Schutzschicht.
In einer anderen Ausführung haben die Systeme die Form eines flachen Beutels, wobei die Rückseite des Beutels für den Wirkstoff undurchlässig ist und die Kontaktseite des Beutels zur Haut aus einer durchlässigen Membran gebildet wird. Der Wirkstoff befindet sich in einem solchen System in Form einer flüssigen oder halbfesten Zubereitung im Beutel. Vorteilhaft ist die Membran mit einer Kleberschicht zur Befestigung auf der Haut versehen. Auch bei dieser Ausführung entsprechend Fig.2 müssen zumindest die klebenden Areale vor Gebrauch mit einer ablösbaren Schutzfolie abgedeckt sein. Damit die Schutzfolie ohne großen Kraftaufwand und ohne Beschädigung des Systems von diesem abgelöst werden kann, muß sie zumindest auf der Kontaktseite zum Pflaster mit einer speziellen Beschichtung versehen sein, die aufgrund ihrer abhäsiven Eigenschaft dafür sorgt, daß die Trennkräfte zwischen Kleberschicht und Schutzfolie für den praktischen Gebrauch nicht zu groß sind. Für alle Kleber mit Ausnahme von Silikonklebern sind deshalb dünne Silikonschichten üblich, die in einem speziellen Verfahren auf ein geeignetes Folienmaterial aufgebracht werden. Generell verwendbar, für Silikonkleber jedoch obligatorisch, sind spezielle Fluorsilikonschichten oder fluorhaltige Polymere. An das Folienmaterial für wirkstoffhaltige Pflastersysteme werden darüber hinaus spezielle Anforderungen gestellt. Da bei allen diesen Systemen auch wirkstoffhaltige Teile in Kontakt mit dem Folienmaterial kommen, muß dieses Material für alle wichtigen Bestandteile inert sein. Dies gilt besonders für den Wirkstoff selbst, aber auch für permeationsverbessernde Substanzen, die in vielen Fällen notwendig sind, um die Barrierewirkung der menschlichen Haut zu beeinflussen, sowie auch für Weichmacher, die in den Kleber eingearbeitet sind und einen Einfluß auf das Klebeverhalten der Systeme auf der Haut haben.

Als Standardmaterial für die Schutzfolie wird deshalb bei praktisch allen Pflastersystemen Polyester (Polyethylentherephthalat) eingesetzt. Für Silikonkleber wird in einigen Fällen auch eine Folie aus fluorhaltigen Polymeren verwendet und so eine zusätzlich abhäsive Beschichtung eingespart. Diese Materialien haben praktisch kein Aufnahmevermögen für Substanzen, die üblicherweise als Bestandteil von wirkstoffhaltigen Pflastersystemen Verwendung finden. Materialien wie Polyethylen, Polypropylen oder Papier sind in vielen Fällen wegen ihres Aufnahmevermögens für solche Inhaltsstoffe der Systeme nicht geeignet. Für die praktische Handhabung, aber auch aus fertigungstechnischen Gründen, müssen Schutzfolien eine Mindestdicke von etwa 50 µm haben. Dies ist deshalb von Nachteil, weil Polyester im Vergleich zu anderen oben erwähnten Materialien ein relativ teures Material darstellt. Dies gilt insbesondere für topische Pflastersysteme, die eine Größe von 300 cm² und mehr erreichen können.

Es ist deshalb Aufgabe der Erfindung, eine verbesserte Schutzfolie für wirkstoffhaltige, insbesondere selbstklebende Pflastersysteme anzugeben, welche bei genügender mechanischer Festigkeit für Wirk- und Hilfsstoffe undurchlässig ist und die Kosten für die bisher verwendeten Schutzfolien aus Polyester deutlich reduziert.

Die Lösung der Aufgabe gelingt mit der Erfindung in überraschender Weise dadurch, daß die Schutzfolie ein Laminat aus einer Papierschicht und einer abhäsiv beschichteten 5 bis 50 µm dicken Polyesterfolie ist.
Der Zusammenhalt der beiden Schichten wird dabei gewährleistet durch die Verwendung von geeigneten Klebern. Durch entsprechende Stabilitätsuntersuchungen mit wirkstoffhaltigen Pflastersystemen konnte gezeigt werden, daß selbst sehr dünne - und deshalb für die Herstellung von wirkstoffhaltigen Pflastern und deren praktischen Gebrauch zu dünne - Polyesterfolien, auf Papier kaschiert, zuverlässig verhindern, daß Inhaltsstoffe der Systeme in das wenig inerte Papier einwandern. Die Papierschicht dient lediglich zur mechanischen Verstärkung der Polyesterfolie und kommt dabei in keinen Kontakt mit Teilen des Systems.

Dabei ist vorgesehen, daß die Polyesterfolie des Laminats auf ihrer Kontaktseite zum Pflastersystem mit einer für den jeweiligen Kleber abhäsiven Beschichtung versehen ist. Bevorzugte Ausgestaltungen der Erfindung sehen vor, daß die Polyesterfolie eine Dicke zwischen 5 und 30 µm, bevorzugt zwischen und 10 und 20 µm aufweist und das Flächengewicht der Papierschicht 30 bis 150, bevorzugt 60 bis 120 g/m² beträgt, wobei die Gesamtdicke mindestens 50 µm betragen muß.
Weitere Ausgestaltungen der Schutzfolie sind entsprechend den Unteransprüchen vorgesehen.

Beispielhafte Ausführungen von Pflastersystemen sind in den Figuren 1-3 dargestellt. Es zeigen:
Fig. 1: im Schnitt ein mehrschichtiges Pflastersystem mit einer ablösbaren Schutzschicht
Fig. 2: im Schnitt ein anderes, mehrschichtiges Pflastersystem mit einer ablösbaren Schutzschicht
Fig. 3: im Schnitt die erfindungsgemäße Ausbildung der ablösbaren Schutzschicht.

Das Pflastersystem gemäß Fig. 1 umfasst eine wirkstoffundurchlässige Rückschicht (1), darunter befindet sich eine selbstklebende, wirkstoffhaltige Matrixschicht (2), die hautseitig mit einer ablösbaren Schutzschicht (3) abgedeckt ist.
Fig. 2 zeigt ein Pflastersystem mit einem andersartigen Aufbau. Dieses weist unterhalb einer für Wirk- und Hilfsstoffe undurchlässigen Rückschicht (1) ein wirkstoffhaltiges Flüssigreservoir (4) mit einer Steuermembran (5) auf, der eine Hautkontaktkleberschicht (6) zugeordnet und die mit einer ablösbaren Schutzschicht (3) abgedeckt ist.

Fig. 3 zeigt die erfindungsgemäße Ausbildung der Schutzschicht (3) in Form eines Laminats, das aus einer abhäsiv beschichteten Polyesterfolie (8) und einer Papierfolie (7) besteht.
Die beiden Folien (7) und (8) sind durch Verwendung geeigneter Kleber zusammengehalten.

Bezüglich der mechanischen Eigenschaften des Laminats (3) zeigten sich in entsprechenden Produktionsversuchen keinerlei Nachteile im Vergleich zu erheblich dickeren Polyesterfolien. Die verwendete Polyesterfolie (8) kann zusätzlich zur abhäsiven Ausrüstung auf einer oder beiden Seiten zusätzlich aluminisiert sein. Diese Aluminisierung geschieht durch Aufdampfen des Aluminiums im Vakuum und hat den Vorteil, daß die abhäsive Ausrüstung bzw. der Kleber, der die Polyesterfolie (8) mit der Papierschicht (7) verbindet, besser auf Aluminium als auf Polyester selbst haftet. Die Dicke der Polyesterfolie (8) kann zwischen 5 und 50 µm liegen. Die Dicke der Papierschicht (7) muß sich nach den jeweiligen Gegebenheiten richten. Die Tabellen 1, 2 und 3 zeigen die Ergebnisse aus Stabilitätsstudien mit zwei unterschiedlichen topischen Systemen mit den Wirkstoffen Ketoprofen, Ibuprofen und Ethylenglykolmonosalicylat. Die Werte in Tabelle 1 und 2 belegen, daß der Gehalt der Pflaster über mindestens zwei Jahre unverändert geblieben ist. Tabelle 3 zeigt die Mengen an Ethylenglykolmonosalicylat, die nach zwei Jahren Lagerung der Pflaster in einer Schutzfolie gemäß der Erfindung gefunden wurden und vergleicht sie mit den Mengen, die bei gleichen Lagerbedingungen in eine handelsüblichen Schutzfolie auf der Basis eines Laminats aus Papier und silikonisiertem Polyethylen eingewandert waren.

Die Ergebnisse der Stabilitätsstudien belegen, daß das der Erfindung zugrundeliegende Laminat (3) aus Papier - (7) und Polyesterfolie (8) allen Anforderungen bezüglich der Stabilität genügt und dem im Handel befindlichen Laminat aus Papier und Polyethylenfolie überlegen ist. Es vereint in vorteilhafter Weise die Überlegenheit des Polyesters bezüglich seiner Inertheit mit den ökonomischen Vorteilen von Papierfolien. Bezüglich seiner maschinellen Verarbeitbarkeit gibt es im Vergleich zu reinen Polyesterfolien keinerlei Einschränkungen.

**Tabelle 1**

| Stabilität des Ketoprofengehalts in einer fettsäurehaltigen selbstklebenden Polyacrylatmatrix | | |
|---|---|---|
| Lagerzeit [Monate] | Lager-temperatur [°C] | Ketoprofen-gehalt [mg/cm²] |
| 0 | / | 0,663 |
| 3 | 25 | 0,651 |
| | 31 | |
| | 40 | 0,657 |
| 6 | 25 | 0,674 |
| | 31 | |
| | 40 | 0,644 |
| 12 | 25 | 0,629 |
| 18 | 25 | 0,659 |
| 24 | 25 | 0,663 |

**Tabelle 2**

| Stabilität des Ibuprofengehalts in einer fettsäurehaltigen selbstklebenden Polyacrylatmatrix | | |
|---|---|---|
| Lagerzeit [Monate] | Lager-temperatur [°C] | Ibuprofen-gehalt [mg/cm²) |
| 0 | / | 0,62 |
| 3 | 25 | 0,63 |
| | 31 | |
| | 40 | 0,61 |
| 6 | 25 | 0,63 |
| | 31 | |
| | 40 | 0,61 |
| 12 | 25 | 0,60 |
| 18 | 25 | 0,61 |

**Tabelle 3**

| Einwanderung von Ethylenglykolmonosalicylat aus einer selbstklebenden Polyacrylatformulierung in ein Laminat aus silikonisiertem Polyethylen/Papier und ein Laminat aus 23 µm silikonisiertem Polyester und 80 g Papier bei 25 °C. | | |
|---|---|---|
| Lagerzeit [Monate] | Papier/ Polyethylen | Papier/ Polyester |
| | | |
| 6 | 0,11 mg/ cm² | 0,001 mg/ cm² |
| 9 | 0,14 mg/ cm² | 0,002 mg/ cm² |
| 12 | 0,27 mg/ cm² | 0,002 mg/ cm² |

## Patentansprüche

1. Wirkstoffhaltiges Pflaster system mit einer ablösbaren Schutzfolie, **dadurch gekennzeichnet, daß** die Schutzfolie ein Laminat aus einer Papierschicht und einer abhäsiv beschichteten, 5 bis 50 µm dicken Polyesterfolie ist.

2. Pflastersystem nach Anspruch 1, **dadurch gekennzeichnet, daß** die Polyesterfolie (8) eine Dicke zwischen 10 und 30 µm aufweist.

3. Pflastersystem nach Anspruch 1 oder 2, **dadurch gekennzeichnet, daß** das Flächengewicht der Papierschicht (3) 30 bis 150, bevorzugt 60 bis 120 g/m² beträgt.

4. Pflastersystem nach Anspruch 1, **dadurch gekennzeichnet, daß** die Polyesterfolie (8) auf mindestens einer Seite aluminiumbedampft ist.

5. Pflastersystem nach Anspruch 1, **dadurch gekennzeichnet, daß** die abhäsive Beschichtung aus Silikonpolymeren besteht.

6. Pflastersystem nach Anspruch 1, **dadurch gekennzeichnet, daß** die abhäsive Beschichtung aus fluorhaltigen Polymeren besteht.

7. Pflastersystem nach Anspruch 1, **gekennzeichnet durch** eine Polyesterfolie auf Polyethylenterephthalatbasis.

8. Ablösbare Schutzfolie für wirkstoffhaltige, Pflastersysteme mit diffusiblen Inhaltsstoffen, gebildet durch ein Laminat nach einem der Ansprüche 1 bis 6 aus einer Papierschicht (7) und einer abhäsiv beschichteten 5 bis 50 µm dicken Polyesterfolie (8) auf Polyethylenterephthalatbasis.

## Claims

1. Active ingredient-containing plaster system with a detachable protective film, **characterized in that** said protective film is a laminate of a paper layer and of an abhesively coated, 5 to 50 µm-thick polyester film.

2. Plaster system according to Claim 1, **characterized in that** the polyester film (8) has a thickness between 10 and 30 µm.

3. Plaster system according to Claim 1 or 2, **characterized in that** the weight per unit area of the paper layer (3) is 30 to 150, preferably 60 to 120, g/m².

4. Plaster system according to Claim 1, **characterized in that** the polyester film (8) is aluminized on at least one side.

5. Plaster system according to Claim 1, **characterized in that** the abhesive coating consists of silicone polymers.

6. Plaster system according to Claim 1, **characterized in that** the abhesive coating consists of fluorine-containing polymers.

7. Plaster system according to Claim 1, **characterized by** a polyester film based on polyethylene terephthalate.

8. Detachable protective film for active ingredient-containing plaster systems with diffusible ingredients, formed by a laminate according to any one of claims 1 to 6 of a paper layer (7) and an abhesively coated 5 to 50 µm-thick polyester film (8) based on polyethylene terephthalate.

## Revendications

1. Système d'emplâtre renfermant des matières actives doté d'une feuille protectrice détachable, **caractérisé en ce que** la feuille protectrice est un stratifié constitué d'une couche de papier et d'un film polyester de 5 à 50 µm d'épaisseur revêtu de matière abhésive.

2. Système d'emplâtre selon la revendication 1, **caractérisé en ce que** le film polyester (8) présente une épaisseur comprise entre 10 et 30 µm.

3. Système d'emplâtre selon la revendication 1 ou 2, **caractérisé en ce que** le grammage de la couche de papier (3) est compris entre 30 et 150, de préférence entre 60 et 120 g/m².

4. Système d'emplâtre selon la revendication 1, **caractérisé en ce que** le film polyester (8) est métallisé à l'aluminium sur au moins une face.

5. Système d'emplâtre selon la revendication 1, **caractérisé en ce que** le revêtement abhésif est constitué de polymères silicones.

6. Système d'emplâtre selon la revendication 1, **caractérisé en ce que** le revêtement abhésif est constitué de polymères fluorés.

7. Système d'emplâtre selon la revendication 1, **caractérisé par** un film polyester à base de térephthalate de polyéthylène.

8. Feuille protectrice détachable pour systèmes d'emplâtre renfermant des matières actives avec des constituants diffusables, formés par un stratifié selon l'une des revendications 1 à 6 à partir d'une couche de papier (7) et d'un film polyester de 5 à 50 µm d'épaisseur revêtu de matière abhésive à base de térephthalate de polyéthylène.
